(19) Europäisches Patentamt
European Patent Office
Office européen des brevets

(11) EP 1 370 241 B1

(12) EUROPEAN PATENT SPECIFICATION

(45) Date of publication and mention of the grant of the patent:
**21.11.2012 Bulletin 2012/47**

(21) Application number: **02701793.8**

(22) Date of filing: **27.02.2002**

(51) Int Cl.:
***A61K 31/215*** (2006.01) ***A61K 9/113*** (2006.01)
***A61K 31/201*** (2006.01) ***A61K 31/203*** (2006.01)
***A61K 31/573*** (2006.01) ***A61K 31/07*** (2006.01)
***A61K 31/355*** (2006.01) ***A61K 8/63*** (2006.01)
***A61K 8/06*** (2006.01) ***A61K 8/67*** (2006.01)

(86) International application number:
**PCT/KR2002/000333**

(87) International publication number:
**WO 2002/067900 (06.09.2002 Gazette 2002/36)**

(54) **MULTI-LAMELLAR EMULSION(MLE) FOR STABILIZING DERMATOLOGICALLY USEFUL INGREDIENTS AND EXTERNAL BASE PREPARATIONS FOR GENERAL SKIN DISEASES UTILIZING THE SAME**

MULTILAMELLARE EMULSION (MLE) ZUR STABILISIERUNG DERMATOLOGISCH NÜTZLICHER INHALTSSTOFFE UND EXTERNER BASENZUBEREITUNGEN FÜR ALLGEMEINE HAUTERKRANKUNGEN

EMULSION MULTI-LAMELLAIRE PERMETTANT LA STABILISATION D'INGREDIENTS DERMATOLOGIQUEMENT ACTIFS, ET PREPARATIONS DE BASE A USAGE EXTERNE POUR DERMATOSES GENERALES

(84) Designated Contracting States:
**DE FR GB IT**

(30) Priority: **28.02.2001 KR 2001010460**

(43) Date of publication of application:
**17.12.2003 Bulletin 2003/51**

(73) Proprietor: **Neopharm Co., Ltd.**
**Daejeon-shi, 305-333 (KR)**

(72) Inventors:
- **PARK, Byeong-deog**
  **Chogju-shi 361-150 (KR)**
- **YOUM, Jong-kyung**
  **Daejeon-shi 306-758 (KR)**
- **SHIM, Jae-dong**
  **Daejeon-shi 305-330 (KR)**
- **JEONG, Se-kyoo**
  **Seoul 156-030 (KR)**
- **KIM, Yang-hee**
  **Seoul 143-917 (KR)**
- **LEE, Seung-hun**
  **Seoul 135-110 (KR)**

(74) Representative: **Humphreys, Ceris Anne et al**
**Abel & Imray**
**20 Red Lion Street**
**London WC1R 4PQ (GB)**

(56) References cited:
**EP-A- 0 875 232** **WO-A-88/06882**
**WO-A-98/21176** **KR-A- 2000 055 082**
**US-A- 4 999 348** **US-A- 5 206 020**
**US-A- 5 980 917** **US-A- 5 985 177**

- **DATABASE CAPLUS [Online] Retrieved from STN Database accession no. 2002:312162**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 370 241 B1

## Description

### Technical Field

The present invention relates to a multi-lamellar emulsion (MLE) for stabilizing dermatologically useful ingredients and an external base preparation for general skin disease utilizing the same, and more particularly, to a MLE having an optimized oil composition for stabilizing dermatologically useful oleophilic ingredients and maintaining the shape of the MLE in case of containing pharmaceutical ingredients such as steroids, and an external base preparation containing the dermatologically useful oleophilic ingredients.

### Background Art

In general, dermatologically useful oleophilic ingredients, including retinol, unsaturated fatty acids, tocopherol and derivatives thereof, oil soluble pigment, vitamin F, etc. are widely used for skin improvement and treatment. Unfortunately, these dermatologically useful oleophilic ingredients readily become unstable in an aqueous solution or under exposure to the air. Hence stabilization of the dermatologically useful oleophilic ingredients is the primary issue in regard to cosmetics and external preparations for skin improvement and treatment, many approaches for stabilizing the dermatologically useful oleophilic ingredients have been contrived.

The methods for stabilizing dermatologically useful oleophilic ingredients are largely divided into the encapsulation method and the method for utilizing the emulsified formulation. The encapsulation method contributes to stabilization of the dermatologically useful oleophilic ingredients by hardening or coating the ingredients by using collagen, alginate, agar, gelatin, chitin or chitosan derivatives to form a primary or secondary capsule and thereby protecting the ingredients against air or water. The method for utilizing the emulsified formulation includes a method of using BHT, tocopherol acetate and EDTA chelate in the simple oil in water (o/w) type or water in oil (w/o) type formulation, a method of using a w/o/w type or o/w/o type multiple emulsion, and a method of delivering the ingredients in the unit of multi-lamella vesicle (MLV) using lecithin. The encapsulation method provides a relatively high stability to the dermatologically useful oleophilic ingredients but has drawbacks such as unpleasant feeling in use and a need of breaking capsules. On the other hand, the method of using the emulsified formulation provides expediency and pleasant feeling in use but poor stability.

On the other hand, external base preparations include a variety of pharmaceutical preparations such as plaster, lotion, liniment, powder, liquid formulation, ointment, emulsion, suspension, cream, cataplasma, paste, and the like. The representative examples of the external base preparations are emulsion preparations such as lotion, ointment, emulsion, and cream.

These emulsion preparations differ greatly in skin penetration of pharmaceutical ingredients depending on the type of base, the emulsion type and the applicability.

Examples of the base may include wax and solid oil ingredients, oils, surfactant, moisturizing agent, thickener, purified water and alcohol, and additionally, perfume, pigment, preservative, organic acid, alkali, UV-absorber, antioxidant, sequesterer, animal/plant extracts, or the like. Among these bases, lipid bases are particularly important and greatly affected by wax and solid oil ingredients and oils. Lipid base, also known as hydrophobic base, is classified into mineral, animal and plant origin lipid bases according to its origin. The lipid base causes no skin irritations and provides skin coating, protection and softening effects due to formation of an airtight oiled layer. The mineral origin lipid base is good in the durability of the airtight oiled layer without a moisturizing function. Specific examples of the mineral origin lipid base may include vaseline and paraffin. The animal/plant origin lipid base is inferior in the durability of the airtight oiled layer to the mineral origin lipid base but has a moisturizing function. Specific examples of the animal/plant origin lipid base may include olive oil, castor oil, and lard. In addition, a silicon-based base can be used.

There are largely two emulsion types, i.e., water-in-oil (W/O) emulsion and oil-in-water (O/W) emulsion. W/O emulsion is divided into a water-containing W/O emulsion and a water-free W/O emulsion. The water-free W/O emulsion is dermatropic and extremely penetrative to the skin, while the water-containing W/O emulsion is excellent in spreadability and applicability to the skin but disadvantageous in regard to the stability of ingredients in water or possibility of contamination with germs. The O/W emulsion is greatly spreadable and particularly excellent in skin penetration of pharmaceutical ingredients. Also, the O/W emulsion has a skin cooling effect due to vaporization of water and may contain various dermatropic aqueous ingredients. But the O/W emulsion is inferior in skin protection function to the other bases.

The emulsion preparations are differently applicable depending on the emulsion type and have a great effect on the skin penetration of the pharmaceutical ingredients. For example, the lotion preparation can be applied onto a large area of the skin due to its good spreadability and the cream preparation can be spread thick on a small area of the skin to apply a large amount of pharmaceutical ingredients.

Specific examples of the pharmaceutical ingredients used for the emulsion base may include steroids, antimicrobials, antifungals and antivirals, and additionally, alpha-hydroxyacid, sun-screening agent, benzoyl peroxide, trenchinoine, anthraline, calcipotriol, etc. Among these ingredients, steroids are most typically used.

Steroids are still widely used for treatment of general dermatitis due to its great efficacy but require cautions in usage because they have adverse side effects, examples of which may include skin deterioration and shrinkage, vasodilation, rebound effect due to interruption of administration, increase in tolerance to steroids, striae distensae(Sort of skin chapping) steroidal acne and rosacea, perioral dermatitis, hyperbrichosis, or the like. The steroids, which require cautions in usage, are still needed to minimize their adverse side effects depending on the features of the base used.

Other pharmaceutical ingredients as used for external base preparations may have a bad side effect such as skin deterioration, dry skin or itching, so that the preparations are needed to have a skin protection effect.

EP0875232 discloses a lipid composition containing a liquid crystal phase. The production of oily micelles dispersed in an aqueous phase is disclosed.

US5985177 discloses oil-in-water-in-oil emulsion compositions.

US5980917 discloses an oil-in-water type cosmetic composition containing retinoids stabilised within the core of a liquid crystal formed by a surfactant.

The English-language abstract of Korean patent no. 2000055082 discloses a cosmetic base of the multi-lamellar emulsion type comprising ceramide-like species, designed for delivering sufficient moisture to the skin and for keeping the skin slightly wet at all times.

**Disclosure of invention**

It is, therefore, a first object of the present invention to provide a multi-lamellar emulsion (MLE) having an optimized oil composition as a base for stabilising dermatologically useful oleophilic ingredients that provides pleasant feeling in use and high stability and can be prepared in a simple way.

It is a second object of the present invention to provide a multi-lamellar emulsion as an external base preparation on the skin for general skin disease according to the first object of the present invention that effectively applies a small amount of pharmaceutical ingredients such as steroids onto the skin and minimises the unfavourable side effects of the pharmaceutical ingredients.

It is a third object of the present invention to provide an external base preparation for skin improvement and treatment using the multi-lamellar emulsion for stabilising dermatologically useful oleophilic ingredients according to the first and second objects of the present invention.

To achieve the above objects of the present invention, there is provided a multi-lamellar emulsion (MLE) for stabilising a dermatologically useful oleophilic ingredient, the MLE comprising:

a skin-lipid based component comprising a lipid ingredient between horny cells,
an oil-based component, and
an emulsifier-based component being a non-ionic surfactant wherein the MLE has an oil composition represented by Equation 1:

[Equation 1]

$$\text{Oil composition} = \frac{\text{Content of oil-based component}}{\text{Content of emulsifier-based component} + \text{Content of skin lipid-based component}} = 0.3 - 1.0$$

wherein the skin lipid-based component is the composition comprising:
a pseudoceramide or natural ceramide
a cholesterol
stearic acid and
cetostearyl alcohol,
wherein the MLE comprises:

(1) 0.5wt% pseudoceramide PC-9S, 0.25wt% cholesterol, 1.0wt% stearic acid, 2.0wt% cetostearyl alcohol, 3.0wt% polyoxyethylene monostearate, 2.0wt% glyceryl monostearate, 5.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or

(2) 0.8wt% pseudoceramide PC-9S, 0.4wt% cholesterol, 2.8wt% stearic acid, 2.0wt% cetostearyl alcohol, 3.0wt% polyoxyethylene glyceryl monostearate, 1.0wt% glyceryl monostearate, 5.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or

(3) 1.0wt% pseudoceramide PC-9S, 0.1wt% cholesterol, 3.5wt% stearic acid, 1.6wt% cetostearyl alcohol, 2.2wt% polyoxyethylene sorbitan monostearate, 0.5wt% sorbitan monostearate, 1.2wt% glyceryl monostearate, 5.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or

(4) 0.4wt% pseudoceramide PC-9S, 0.15wt% cholesterol, 1.0wt% stearic acid, 2.0wt% cetostearyl alcohol, 2.0wt% polyoxyethylene glyceryl monostearate, 1.0wt% glyceryl monostearate, 4.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or

(5) 0.5wt% pseudoceramide PC-9S, 0.15wt% cholesterol, 1.2wt% stearic acid, 2.5wt% cetostearyl alcohol, 2.5wt% polyoxyethylene glyceryl monostearate, 1.2wt% glyceryl monostearate, 3.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or

(6) 0.6wt% pseudoceramide PC-9S, 0.2wt% cholesterol, 1.5wt% stearic acid, 3.0wt% cetostearyl alcohol, 3.0wt% polyoxyethylene glyceryl monostearate, 1.5wt% glyceryl monostearate, 5.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or

(7) 0.8wt% pseudoceramide PC-9S, 0.3wt% cholesterol, 1.8wt% stearic acid, 3.5wt% cetostearyl alcohol, 3.2wt% polyoxyethylene glyceryl monostearate, 1.8wt% glyceryl monostearate, 8.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or

(8) 1.0wt% pseudoceramide PC-9S, 0.3wt% cholesterol, 2.0wt% stearic acid, 4.0wt% cetostearyl alcohol, 4.0wt% polyoxyethylene glyceryl monostearate, 2.0wt% glyceryl monostearate, 13.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or

(9) 0.6wt% pseudoceramide PC-9SP, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.5wt% hydrocortisone, 0.3wt% preservative, 0.2wt% carboxy vinyl polymer and water; or

(10) 0.6wt% pseudoceramide PC-9SP, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.12wt% betamethasone valerate, 0.3wt% preservative, 0.2wt% carboxy vinyl polymer and water; or

(11) 0.6wt% pseudoceramide PC-9SP, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.05wt% clobetasol proprionate, 0.3wt% preservative, 0.2wt% carboxy vinyl polymer and water; or

(12) 0.6wt% pseudoceramide PC-9SP, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 1.0wt% tretinoin, 0.3wt% preservative, 0.2wt% carboxy vinyl polymer and water; or

(13) 0.6wt% pseudoceramide PC-9SP, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.5wt% linoleic acid, 0.3wt% preservative, 0.2wt% carboxy vinyl polymer and water; or

(14) 0.6wt% ceramide III, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.5wt% hydrocortisone, 0.3wt% preservative, 0.2wt% carboxy vinyl polymer and water; or

(15) 0.6wt% ceramide III, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.12wt% betamethasone valerate, 0.3wt% preservative, 0.2wt% carboxy vinyl polymer and water; or

(16) 0.6wt% ceramide III, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.05wt% clobetasol proprionate, 0.3wt% preservative, 0.2wt% carboxy vinyl polymer and water; or

(17) 0.6wt% ceramide III, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 1.0wt% tretinoin, 0.3wt% preservative, 0.2wt% carboxy vinyl polymer and water; or

(18) 0.6wt% ceramide III, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.5wt% linoleic acid, 0.3wt% preservative, 0.2wt% carboxy vinyl polymer and water.

## Brief Description of the Drawings

FIG. 1 is a three-phase (emulsifier-oil-skin lipid) diagram for preparing a multi-lamellar emulsion having an optimal oil composition according to the present invention; and

FIGS. 2a and 2b are polarized microscopic pictures showing the lamellar structure of an external preparation using the multi-lamellar emulsion according to the present invention, in which FIGS. 2a and 2b show the excellent ageing stability of the lamellar structure after four weeks at 25°C and 45°C, respectively.

FIG. 3 is a polarized microscope picture showing that a base for the external base preparation of the present invention containing pharmaceutical ingredients has a lamellar structure of MLE;

FIG. 4 is a transmission electron microscope picture of a base for the external base preparation of the present invention containing pharmaceutical ingredients;

FIGS. 5a and 5b are graphs showing the appliability of a base for the external base preparation of the present invention as a MLE containing steroids as pharmaceutical ingredients, in which a measurement of the transepidermal water loss (TEWL) of a nude mouse evaluated as an index of skin deterioration shows that the MLE containing steroids maintains the normal condition of the skin and that the MLE without steroids causes skin deterioration as an adverse side effect; and

FIGS. 6a and 6b are graphs showing the applicability of a base for the external base preparation of the present invention as a MLE containing steroids as pharmaceutical ingredients, in which a measurement of the skin hydration of a nude mouse evaluated as an index of skin deterioration shows that the MLE containing steroids maintains the normal moisturizing ability of the skin and that the MLE without steroids causes a deterioration of the moisturizing ability of the skin as an adverse side effect.

## Best Mode for Carrying Out the Invention

Hereinafter, a description will be given as to a method for preparing an MLE having a predetermined oil composition according to the present invention.

The MLE preparation method includes melting pseudoceramide, natural ceramide, cholesterol, higher fatty acid, higher fatty alcohol, polyalcohol or any mixture thereof together with general oils and a non-ionic surfactant, uniformly mixing these components, adding a warm purified water to the mixture to cause phase inversion emulsification, adding a thickener immediately after the emulsification and uniformly mixing these components, and cooling the resulting mixture at the room temperature. The added amount of the purified water is, if not specifically limited to, 50 to 95 wt.%, and preferably 65 to 85 wt.% based on the total weight of the MLE.

On the other hand, optimization of the oil composition of the MLE involves drawing up a three-phase diagram shown in FIG. 1; preparing a skin lipid phase comprising pseudoceramide, natural ceramide, cholesterol, higher fatty acid, higher fatty alcohol, or any mixture thereof, and an emulsifier phase comprising a non-ionic surfactant to form a lipid-emulsion system in which the MLE will be formed; and adding different oils to regulate the oil composition.

Hereinafter, the present invention will be described in detail by way of the following examples and experimental examples.

<u>Examples 1 to 8 and Comparative Examples 1 to 4</u>

Pseudoceramide PC-9S, cholesterol, higher fatty acid, non-ionic surfactant, higher fatty alcohol, polyalcohol, thickener and purified water were mixed with the composition presented in Table 1 to prepare a multi-lamellar emulsion (MLE). Retinol palmitate was used as an indicator of the dermatologically useful oleophilic ingredient. Various MLE's were prepared depending on the type of the emulsifier (in Examples 1 to 4) or the oil composition (in Examples 5 to 8). The MLE's of Comparative Examples 1 and 2 didn't contain pseudoceramide, cholesterol or fatty acid as an intercellular lipid ingredient, the MLE of Comparative Example 3 contained no oil (in Comparative Example 3), and the MLE of Comparative Example 4 was scarcely formed due to the excessively high oil composition.

The pseudo-ceramide PC-9S had the above-mentioned formula (1), in which $R_1$ is $-C_{15}H_{31}$ or $C_{17}H_{35}$; and $R_2$ is $-C_{14}H_{29}$ or $C_{16}H_{33}$. The thickener was carboxy vinyl polymer.

Table 1

| Ingredient | A1 | A2 | A3 | A4 | A5 | A6 | A7 | A8 | B1 | B2 | B3 | B4 |
|---|---|---|---|---|---|---|---|---|---|---|---|---|
| Pseudo-ceramide | 0.5 | 0.8 | 1.0 | 0.4 | 0.5 | 0.6 | 0.8 | 1.0 | - | - | 1.0 | 1.0 |
| Natural ceramide III | - | - | - | - | - | - | - | - | 0.3 | - | - | - |
| Cholesterol | 0.25 | 0.4 | 0.1 | 0.15 | 0.15 | 0.2 | 0.3 | 0.3 | - | - | 0.3 | 0.3 |
| Stearic acid | 1.0 | 2.8 | 3.5 | 1.0 | 1.2 | 1.5 | 1.8 | 2.0 | - | 1.6 | 2.0 | 2.0 |
| Cetostearylacohol | 2.0 | 2.0 | 1.6 | 2.0 | 2.5 | 3.0 | 3.5 | 4.0 | 3.5 | 1.4 | 4.0 | 4.0 |
| POE mono-stearate | 3.0 | - | - | - | - | - | - | - | - | - | - | - |
| POE sorbitan mono-stearate | - | - | 2.2 | - | - | - | - | - | - | - | - | - |
| Sorbitan mono-stearate | - | - | 0.5 | - | - | - | - | - | - | - | - | - |
| POE glyceryl mono-stearate | - | 3.0 | - | 2.0 | 2.5 | 3.6 | 3.2 | 4.0 | 2.0 | 2.0 | 4.0 | 4.0 |
| Glyceryl mono-stearate | 2.0 | 1.0 | 1.2 | 1.0 | 1.2 | 1.5 | 1.8 | 2.0 | 1.0 | 1.0 | 2.0 | 2.0 |
| Oils Olive oil Liquid paraffin Vaseline Paraffin Wax | 5.0 | 5.0 | 5.0 | 4.0 | 3.0 | 5.0 | 8.0 | 13.0 | 5.0 | 4.0 | 0.0 | 20.0 |
| 1,3-butylene glycol | 5.0 | 5.0 | 5.0 | 5.0 | 5.0 | 5.0 | 5.0 | 5.0 | 5.0 | 5.0 | 5.0 | 5.0 |
| Retinol palmitate | 0.3 | 0.3 | 0.3 | 0.3 | 0.3 | 0.3 | 0.3 | 0.3 | 0.3 | 0.3 | 0.3 | 0,3 |
| Thickener | 0.2 | 0.2 | 0.2 | 0.2 | 0.2 | 0.2 | 0.2 | 0.2 | 0.2 | 0.2 | 0.2 | 0.2 |
| Purified water | dregs | dregs | dregs | dregs | dregs | dregs | dregs | dregs | dregs | dregs | dregs | dregs |
| Oil composition | 0.57 | 0.5 | 0.5 | 0.61 | 0.37 | 0.55 | 0.70 | 0.99 | 0.75 | 0.67 | 0 | 1.50 |

Note) A: Example
B: Comparative Example
Oil composition = content of oil-based component / (content of emulsifier-based component + content of skin lipid-based component)

Experimental Example 1: Thermal Stability Test

A four-week thermal stability test was performed on the MLE's prepared in Examples 1 to 8 and Comparative Examples 1 to 4 in order to measure the stability of retinol palmitate. After 4 weeks at 25 °C and 45 °C, high-performance liquid chromatography (HPLC) was utilized to analyze stable retinol palmitate. The results are presented in Table 2.

Table 2

| Temp / Term | A1 | | A2 | | A3 | | A4 | | B1 | | B2 | |
|---|---|---|---|---|---|---|---|---|---|---|---|---|
| | 25° C | 45° C | 25° C | 45° C | 25° C | 45° C | 25° C | 45° C | 25° C | 45° C | 25° C | 45° C |
| After 2 weeks (%) | 95.3 | 68.9 | 94.2 | 71.2 | 91.2 | 72.3 | 93.3 | 68.3 | 72.3 | 60.2 | 68.9 | 58.6 |
| After 4 weeks (%) | 92.5 | 59.5 | 91.2 | 67.4 | 89.3 | 58.2 | 88.9 | 59.3 | 54.3 | 45.3 | 53.2 | 42.5 |

| Temp / Term | A5 | | A6 | | A7 | | A8 | | B3 | | B4 | |
|---|---|---|---|---|---|---|---|---|---|---|---|---|
| | 25° C | 45° C | 25° C | 45° C | 25° C | 45° C | 25° C | 45° C | 25° C | 45° C | 25° C | 45° C |
| After 2 weeks (%) | 99.8 | 73.0 | 98.9 | 75.9 | 95.9 | 82.3 | 94.9 | 77.2 | 73.1 | 71.2 | 89.7 | 62.3 |
| After 4 weeks (%) | 97.2 | 60.7 | 95.4 | 65.8 | 94.6 | 67.3 | 93.0 | 66.3 | 64.3 | 23.9 | 74.2 | 48.2 |

Note) A: Example

B: Comparative Example

As is apparent from the results of Table 2 (Examples 5 to 8), the stability of retinol palmitate was increased with an increase in the oil composition. However, for Examples 7 and 8, the stability of retinol palmitate was lower at the oil composition of about 1.0 than at the oil composition of about 0.7 (Example 7). This means that there is an optimal oil composition in the MLE of the present invention. Also, as is seen from the results of Examples 1 to 4 and Comparative Examples 1 and 2, the stability of retinol palmitate was low without a proper mixture of horny intercellular lipid gradients, cholesterol and fatty acid even though the oil composition was within the range of 0.3 to 1.0.

Experimental Example 2: Stability Test of External MLE Preparation

To measure the stability of the lamellar structure of the external MLE preparation containing retinol palmitate, the preparation of Example 3 was placed at 25 °C or 45 °C for 4 weeks and observed with a polarized microscope (Optiphotpol-2, Nikon Company) whether the maltese cross peculiar to the MLE appeared. The results are presented in FIG. 2.

As is apparent from FIG. 2, the lamellar structure of the MLE according to the present invention was stable after 4 weeks at 45 °C (in FIG. 2b) as well as after 4 weeks at 25 °C (in FIG. 2a). Such a high stability contributes to effective stabilization of various dermatologically useful oleophilic ingredients such as retinol palmitate.

Experimental Example 3: Skin Irritation Test

To measure the skin Irritation ability of the external MLE preparation of the present invention, 30 healthy adult persons had the lower arm onto which Haye's chambers stained with a predetermined amount (about 2.0 g), which is used for the patch test, of the individual preparations of Example 6 and Comparative Examples 1 and 4 were applied for 48 hours. After an elapse of 30 minutes to one hour since the Haye's chambers were removed, the change of the complexion was observed with unaided eyes for the primary finding, and again after more 48 hours for the secondary finding.

As is apparent from the results in Table 3, the preparation of Example 6 less Irritated the skin than those of Comparative Examples 1 and 4.

Table 3

| Sample | Number of Testees | Results | | | | | | | | | | Degree of stimuli |
|---|---|---|---|---|---|---|---|---|---|---|---|---|
| | | Non | | Uncertain | | Slightly | | Yes | | Severe | | |
| | | I | II | I | II | I | II | I | II | I | II | |
| Example 6 | 30 | 30 | 30 | | | | | | | | | |
| Comparative Example 1 | 30 | 25 | 30 | 5 | | | | | | | | |
| Comparative Example 4 | 30 | 24 | 28 | 1 | 0 | 5 | 2 | | | | | |

Examples 9 to 18 and Comparative Examples 5 and 6

Natural and pseudo- ceramides, cholesterol, higher fatty acid, oils, non-ionic surfactant, higher fatty alcohol, polyalcohol, thickener and purified water were mixed with the composition presented in the following table to prepare a multi-lamellar emulsion (MLE). Steroid ingredients as used herein were hydrocortisone, Betamethasone Valerate and Clobetasol Proprionate, and non-steroid ingredients as used herein were tretinoin and linoleic acid.

The natural ceramide as used herein was ceramide III (Doosan Biotec, Korea, $C_{36}H_{73}NO_4$) and the pseudoceramide as used herein was PC-9SP (Neo-Pharm, Korea, Pseudoceramide PC-9SP (Mixture of myristyl oxostearamide monoethanol amine, myristyl arachidamide monoethanol amine, palmityl oxostearamide monoethanol amine and palmityl arachidamide monoethanol amine). The thickener as used herein was carboxyvinyl polymer.

Examples 9 to 13 prepared MLEs comprising the pseudoceramide and the pharmaceutical ingredients, and Examples 14 to 18 prepared MLEs comprising the natural ceramide and the pharmaceutical ingredients. Comparative Examples 5 and 6 did not form a MLE but added the pharmaceutical ingredients for the sake of performing a test in comparison with Examples.

[Table]

| Ingredient | A9 | A10 | A11 | A12 | A13 | A14 | A15 | A16 | A17 | A18 | B5 | B6 |
|---|---|---|---|---|---|---|---|---|---|---|---|---|
| PC-9SP | 0.6 | 0.6 | 0.6 | 0.6 | 0.6 | - | - | - | - | - | - | - |
| Ceramide-III | - | - | - | - | - | 0.6 | 0.6 | 0.6 | 0.6 | 0.6 | - | - |
| Cholesterol | 0.2 | 0.2 | 0.2 | 0.2 | 0.2 | 0.2 | 0.2 | 0.2 | 0.2 | 0.2 | - | - |
| Stearic acid | 1.5 | 1.5 | 1.5 | 1.5 | 1.5 | 1.5 | 1.5 | 1.5 | 1.5 | 1.5 | 0.7 | 0.7 |
| Cetoatearyl acohol | 3.5 | 3.5 | 3.5 | 3.5 | 3.5 | 3.5 | 3.5 | 3.5 | 3.5 | 3.5 | 3.5 | 3.5 |
| POE glyceryl mono-stearate | 3.5 | 3.5 | 3.5 | 3.5 | 3.5 | 3.5 | 3.5 | 3.5 | 3.5 | 3.5 | 3.5 | 3.5 |
| Glyceryl mono-stearate | 1.5 | 1.5 | 1.5 | 1.5 | 1.5 | 1.5 | 1.5 | 1.5 | 1.5 | 1.5 | 1.5 | 1.5 |
| 1,3-butylene glycol | 5.0 | 5.0 | 5.0 | 5.0 | 5.0 | 5.0 | 5.0 | 5.0 | 5.0 | 5.0 | 5.0 | 5.0 |
| Glycerin | 5.0 | 5.0 | 5.0 | 5.0 | 5.0 | 5.0 | 5.0 | 5.0 | 5.0 | 5.0 | 5.0 | 5.0 |
| Oils Squalane Macademia nut oil Olive oil Oliwax | 5.0 | 5.0 | 5.0 | 5.0 | 5.0 | 5.0 | 5.0 | 5.0 | 5.0 | 5.0 | 5.0 | 5.0 |
| Hydrocortison | 0.5 | .5 | - | - | - | 0.5 | - | - | - | - | 5.0 | - |
| Betamethasone Valerate | - | 0.12 | - | - | - | - | 0.12 | - | - | - | - | - |
| Clobetasol Proprionate | - | - | 0.05 | - | - | - | - | 0.05 | - | - | - | 0.05 |
| Tretinoin | - | - | - | 1.0 | - | - | - | - | 1.0 | - | - | - |
| Linoleic acid | - | - | - | - | 0.5 | - | - | - | - | 0.5 | - | - |
| Preservative | 0.3 | 0.3 | 0.3 | 0.3 | 0.3 | 0.3 | 0.3 | 0.3 | 03 | 0.3 | 0.3 | 0.3 |
| Thickener | 0.2 | 0.2 | 0.2 | 0.2 | 0.2 | 0.2 | 0.2 | 0.2 | 0.2 | 0.2 | 0.2 | 0.2 |
| Purified water | dregs | dregs | dregs | dregs | dregs | dregs | dregs | dregs | dregs | dregs | dregs | Dregs |
| Note) A: Example<br>B: Comparative Example | | | | | | | | | | | | |

Experimental Example 4: Identification of MLE

A polarized microscope (Optiphot-2, Nikon) was used to observe a Maltese cross that identifies the lamellar structure of the MLEs as an external base preparation prepared in Examples 9 to 18 irrespective of the added pharmaceutical ingredients.

According to the microscopic observation, all the MLEs in Examples 9 to 18 had a lamellar structure. For example, a polarized microscope picture of the lamellar structure in Example 13 is shown in FIG. 3. As seen from FIG. 3, the MLE was formed in the case of using the pharmaceutical ingredients. Also, the MLE was formed with a lamellar structure in the case of using the natural ceramide in the preparation of the emulsion.

To identify the formation of the MLE more accurately, the preparations of Example 10 was dyed with ruthnium and observed with a transmission electron microscope (TEM). As shown in FIG. 4, a normal MLE was successfully formed.

Accordingly, the application of the MLE containing pharmaceutical ingredients for general skin disease provides the efficacy of the pharmaceutical ingredients as well as the MLE on the skin.

Experimental Example 5: Stability of MLE

To measure the stability of the lamellar structure of the external base preparation as the MLE containing pharmaceutical ingredients according to the present invention, the samples of Examples 9 to 18 were placed in a freeze-thaw cycle at 25 °C and 45°C and observed with a polarized microscope to determine whether the maltese cross peculiar to the MLE appeared. According to the microscopic observation, all the preparations of Examples 9 to 18 maintained a good lamellar structure of the MLE containing pharmaceutical ingredients. As can be seen from the results, the MLEs were allowed to contain different pharmaceutical ingredients due to high stability.

Experimental Example 6: Skin Barrier Function of MLE

A nude mouse was used to evaluate the skin barrier function of the MLE of the present invention against the adverse side effects of the pharmaceutical ingredients, particularly, steroids.

Generally, the application of steroids onto the skin may cause side effects such as skin deterioration and shrinkage to deteriorate the skin barrier function, as may be seen by the increase in transepidermal water loss (TEWL) (Skin Research and Technology 2001; 7:73-77).

The preparations containing steroids in Examples 9 and 11, and those as prepared in Comparative Examples 5 and 6 were applied onto the skin of the nude mouse twice a day for two weeks and the TEWL measurement was periodically performed with a Tewameter TM210 (C+K electronic, Germany).

To evaluate the moisturizing ability of the skin, a Comeometer (C+K electronic, Germany) was used to measure the change of skin hydration.

As can be seen from the results of FIG. 5, the MLEs of the present invention as prepared in Examples 9 and 11 had no change in the TEWL level. But the samples as prepared in Examples 5 and 6 that did not form MLE caused a significant increase in the TEWL level, showing a deterioration of the skin barrier function.

On the other hand, as shown in FIG. 6, the preparations of Examples 9 and 11 maintained a constant skin hydration level but those of Comparative Examples 5 and 6 had a gradual decrease in the skin hydration level due to the skin damage resulting from the side effect of steroids.

Accordingly, the MLE as a base for external base preparation for treatment of general skin disease according to the present invention can minimize the adverse side effects of the pharmaceutical ingredients such as steroids and provide a dermatologically useful external base preparation.

**Industrial Applicability**

As described above, the external preparation using the MLE having an optimal oil composition according to the present invention for stabilizing dermatologically useful oleophilic ingredients is incorporated with the dermatologically useful oleophilic ingredients which are effectively stabilized, which exhibits excellent appetence to the skin, thereby providing various external preparations for skin improvement and treatment because it hardly irritates the skin. Furthermore, the use of the MLE of the present invention as a base for external base preparations for general skin disease recovers the skin barrier function deteriorated by the pharmaceutical ingredients and minimizes the other adverse side effects of the pharmaceutical ingredients. Furthermore, the MLE as a base for external base preparations according to the present invention is so dermatropic as to effectively apply pharmaceutical ingredients onto the skin and has a high efficacy with a small amount. Accordingly, the MLE of the present invention is very useful as a base for the preparations for treatment of general skin diseases.

**Claims**

1. A multi-lamellar emulsion (MLE) for stabilising a dermatologically useful oleophilic ingredient, the MLE comprising:

a skin-lipid based component comprising a lipid ingredient between horny cells,
an oil-based component, and
an emulsifier-based component being a non-ionic surfactant wherein the MLE has an oil composition represented by Equation 1:

[Equation 1]

$$Oil\,composition = \frac{Content\,of\,oil-based\,component}{Content\,of\,emulsifier-based\,component + Content\,of\,skin\,lipid-based\,component} = 0.3-1.0$$

wherein the skin lipid-based component is the composition comprising:
a pseudoceramide or natural ceramide
a cholesterol
stearic acid and
cetostearyl alcohol,
wherein the MLE comprises:

(1)
0.5wt% pseudoceramide PC-9S, 0.25wt% cholesterol, 1.0wt% stearic acid, 2.0wt% cetostearyl alcohol, 3.0wt% polyoxyethylene monostearate, 2.0wt% glyceryl monostearate, 5.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or
(2)
0.8wt% pseudoceramide PC-9S, 0.4wt% cholesterol, 2.8wt% stearic acid, 2.0wt% cetostearyl alcohol, 3.0wt% polyoxyethylene glyceryl monostearate, 1.0wt% glyceryl monostearate, 5.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or
(3)
1.0wt% pseudoceramide PC-9S, 0.1wt% cholesterol, 3.5wt% stearic acid, 1.6wt% cetostearyl alcohol, 2.2wt% polyoxyethylene sorbitan monostearate, 0.5wt% sorbitan monostearate, 1.2wt% glyceryl monostearate, 5.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water;or
(4)
0.4wt% pseudoceramide PC-9S, 0.15wt% cholesterol, 1.0wt% stearic acid, 2.0wt% cetostearyl alcohol, 2.0wt% polyoxyethylene glyceryl monostearate, 1.0wt% glyceryl monostearate, 4.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or
(5)
0.5wt% pseudoceramide PC-9S, 0.15wt% cholesterol, 1.2wt% stearic acid, 2.5wt% cetostearyl alcohol, 2.5wt% polyoxyethylene glyceryl monostearate, 1.2wt% glyceryl monostearate, 3.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or
(6)
0.6wt% pseudoceramide PC-9S, 0.2wt% cholesterol, 1.5wt% stearic acid, 3.0wt% cetostearyl alcohol, 3.0wt% polyoxyethylene glycerol monostearate, 1.5wt% glyceryl monostearate, 5.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or
(7)
0.8wt% pseudoceramide PC-9S, 0.3wt% cholesterol, 1.8wt% stearic acid, 3.5wt% cetostearyl alcohol, 3.2wt% polyoxyethylene glyceryl monostearate, 1.8wt% glyceryl monostearate, 8.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or

(8)

1.0wt% pseudoceramide PC-9S, 0.3wt% cholesterol, 2.0wt% stearic acid, 4.0wt% cetostearyl alcohol, 4.0wt% polyoxyethylene glyceryl monostearate, 2.0wt% glyceryl monostearate, 13.0wt% of a mixture of olive oil, liquid paraffin, Vaseline and paraffin wax, 5.0wt% 1,3-butylene glycol, 0.3wt% retinol palmitate, 0.2wt% carboxy vinyl polymer and water; or

(9)

0.6wt% pseudoceramide PC-9SP, O.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.5wt% hydrocortisone, 0.3wt% preservative, 0.2wt% carboxy vinyl polymer and water; or

(10)

0.6wt% pseudoceramide PC-9SP, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.12wt% betamethasone valerate, 0.3wt% preservative, 0.2wt% carboxy vinyl polymer and water; or

(11)

0.6wt% pseudoceramide PC-9SP, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.05wt% clobetasol proprionate, 0.3wt% preservative, 0.2wt% carboxy vinyl polymer and water; or

(12)

0.6wt% pseudoceramide PC-9SP, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 1.0wt% tretinoin, 0.3wt% preservative, 0.2wt% carboxy vinyl polymer and water; or

(13)

0.6wt% pseudoceramide PC-9SP, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.5wt% linoleic acid, 0.3wt% preservative, 0.2wt% carboxy vinyl polymer and water; or

(14)

0.6wt% ceramide III, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.5wt% hydrocortisone, 0.3wt% preservative, 0.2wt% carboxy vinyl polymer and water; or

(15)

0.6wt% ceramide III, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.12wt% betamethasone valerate, 0.3wt% preservative, 0.2wt% carboxy vinyl polymer and water; or

(16)

0.6wt% ceramide III, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.05wt% clobetasol proprionate, 0.3wt% preservative, 0.2wt% carboxy vinyl polymer and water; or

(17)

0.6wt% ceramide III, 0.2wt% cholesterol, 1.5wt% stearic acid, 3.5wt% cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 1.0wt% tretinoin, 0.3wt% preservative, 0.2wt% carboxy vinyl polymer and water; or

(18)

0.6wt% ceramide III, 0.2wt% cholesterol, 1.5wt% stearic acid, 3.5wt; cetostearyl alcohol, 3.5wt% polyoxyethylene glyceryl monostearate, 1.5wt% glyceryl monostearate, 5.0wt% 1,3-butylene glycol, 5.0wt% glycerine, 5.0wt% of a mixture of squalane, macadamia nut oil, olive oil and oliwax, 0.5wt% linoleic acid, 0.3wt% preservative, 0.2wt% carboxy vinyl polymer and water.

**Patentansprüche**

1. Multilamellare Emulsion (MLE) zum Stabilisieren eines dezmatologisch nützlichen oleophilen Inhaltsstoffs, wobei die MLE Folgendes aufweist:

   eine auf Hautlipiden basierende Komponente, die einen Lipidbestandteil zwischen hornartigen Zellen aufweist,
   eine auf Öl basierende Komponente und
   eine auf einen Emulgator basierende Komponente, die ein nichtionisches oberflächenaktives Mittel ist,
   wobei die MLE eine Ölzusammensetzung aufweist, die mit der Gleichung 1 angegeben wird:

   [Gleichung 1]

$$\text{Ölzusammensetzung} = \frac{\text{Gehalt der auf Öl basierenden Komponente}}{\text{Gehalt der auf Emulgator basierenden Komponente} + \text{Gehalt der auf Hautlipiden basierenden Komponente}} = 0{,}3 - 1{,}0$$

   wobei die auf Hautlipiden basierende Komponente eine Zusammensetzung ist, die Folgendes aufweist:
   ein Pseudoceramid oder ein natürliches Ceramid
   ein Cholesterin
   Stearinsäure und
   Cetostearylalkohol,
   wobei die MLE Folgendes aufweist:

   (1)
   0,5 Gew.-% Pseudoceramid PC-9S, 0,25 Gew.-% Cholesterin, 1,0 Gew.-% Stearinsäure, 2,0 Gew.-% Cetostearylalkohol, 3,0 Gew.-% Polyoxyethylenmonostearat, 2,0 Gew.-% Glycerylmonostearat, 5,0 Gew.-% eines Gemischs von Olivenöl, flüssigem Paraffin, Vaseline und Paraffinwachs, 5,0 Gew.-% 1,3-Butylenglycol, 0,3 Gew.-% Retinolpalmitat, 0,2 Gew.-% Carboxyvinylpolymer und Wasser; oder
   (2)
   0,8 Gew.-% Pseudoceramid PC-9S, 0,4 Gew.-% Cholesterin, 2,8 Gew.-% Stearinsäure, 2,0 Gew.-% Cetostearylalkohol, 3,0 Gew.-% Polyoxyethylenglycerylmonostearat, 1,0 Gew.-% Glycerylmonostearat, 5,0 Gew.-% eines Gemischs von Olivenöl, flüssigem Paraffin, Vaseline und Paraffinwachs, 5,0 Gew.-% 1,3-Butylenglycol, 0,3 Gew.-% Retinolpalmitat, 0,2 Gew.-% Carboxyvinylpolymer und Wasser; oder
   (3)
   1,0 Gew.-% Pseudoceramid PC-9S, 0,1 Gew.-% Cholesterin, 3,5 Gew.-% Stearinsäure, 1,6 Gew.-% Cetostearylalkohol, 2,2 Gew.-% Polyoxyethylensorbitanmonostearat, 0,5 Gew.-% Sorbitanmonostearat, 1,2 Gew.-% Glycerylmonostearat, 5,0 Gew.-% eines Gemischs von Olivenöl, flüssigem Paraffin, Vaseline und Paraffinwachs, 5,0 Gew.-% 1,3-Butylenglycol, 0,3 Gew.-% Retinolpalmitat, 0,2 Gew.-% Carboxyvinylpolymer und Wasser; oder
   (4)
   0,4 Gew.-% Pseudoceramid PC-9S, 0,15 Gew.-% Cholesterin, 1,0 Gew.-% Stearinsäure, 2,0 Gew.-% Cetostearylalkohol, 2,0 Gew.-% Polyoxyethylenglycerylmonostearat, 1,0 Gew.-% Glycerylmonostearat, 4,0 Gew.-% eines Gemischs von Olivenöl, flüssigem Paraffin, Vaseline und Paraffinwachs, 5,0 Gew.-% 1,3-Butylenglycol, 0,3 Gew.-% Retinolpalmitat, 0,2 Gew.-% Carboxyvinylpolymer und Wasser; oder
   (5)
   0,5 Gew.-% Pseudoceramid PC-9S, 0,15 Gew.-% Cholesterin, 1,2 Gew.-% Stearinsäure, 2,5 Gew.-% Cetostearylalkohol, 2,5 Gew.-% Polyoxyethylenglycerylmonostearat, 1,2 Gew.-% Glycerylmonostearat, 3,0 Gew.-% eines Gemischs von Olivenöl, flüssigem Paraffin, Vaseline und Paraffinwachs, 5,0 Gew.-% 1,3-Butylenglycol, 0,3 Gew.-% Retinolpalmitat, 0,2 Gew.-% Carboxyvinylpolymer und Wasser; oder
   (6)
   0,6 Gew.-% Pseudoceramid PC-9S, 0,2 Gew.-% Cholesterin, 1,5 Gew.-% Stearinsäure, 3,0 Gew.-% Cetostearylalkohol, 3,0 Gew.-% Polyoxyethylenglycerylmonostearat, 1,5 Gew.-% Glycerylmonostearat, 5,0 Gew.-% eines Gemischs von Olivenöl, flüssigem Paraffin, Vaseline und Paraffinwachs, 5,0 Gew.-% 1,3-Butylenglycol, 0,3 Gew.-% Retinolpalmitat, 0,2 Gew.-% Carboxyvinylpolymer und Wasser; oder
   (7)

0,8 Gew.-% Pseudoceramid PC-9S, 0,3 Gew.-% Cholesterin, 1,8 Gew.-% Stearinsäure, 3,5 Gew.-% Cetostearylalkohol, 3,2 Gew.-% Polyoxyethylenglycerylmonostearat, 1,8 Gew.-% Glycerylmonostearat, 8,0 Gew.-% eines Gemischs von Olivenöl, flüssigem Paraffin, Vaseline und Paraffinwachs, 5,0 Gew.-% 1,3-Butylenglycol, 0,3 Gew.-% Retinolpalmitat, 0,2 Gew.-% Carboxyvinylpolymer und Wasser; oder

(8)

1,0 Gew.-% Pseudoceramid PC-9S, 0,3 Gew.-% Cholesterin, 2,0 Gew.-% Stearinsäure, 4,0 Gew.-% Cetostearylalkohol, 4,0 Gew.-% Polyoxyethylenglycerylmonostearat, 2,0 Gew.-% Glycerylmonostearat, 13,0 Gew.-% eines Gemischs von Olivenöl, flüssigem Paraffin, Vaseline und Paraffinwachs, 5,0 Gew.-% 1,3-Butylenglycol, 0,3 Gew.-% Retinolpalmitat, 0,2 Gew.-% Carboxyvinylpolymer und Wasser; oder

(9)

0,6 Gew.-% Pseudoceramid PC-9SP, 0,2 Gew.-% Cholesterin, 1,5 Gew.-% Stearinsäure, 3,5 Gew.-% Cetostearylalkohol, 3,5 Gew.-% Polyoxyethylenglycerylmonostearat, 1,5 Gew.-% Glycerylmonostearat, 5,0 Gew.-% 1,3-Butylenglycol, 5,0 Gew.-% Glycerin, 5,0 Gew.-% eines Gemischs von Squalan, Macadamianussöl, Olivenöl und Oliwax, 0,5 Gew.-% Hydrocortison, 0,3 Gew.-% Konservierungsmittel, 0,2 Gew.-% Carboxyvinylpolymer und Wasser; oder

(10)

0,6 Gew.-% Pseudoceramid PC-9SP, 0,2 Gew.-% Cholesterin, 1,5 Gew.-% Stearinsäure, 3,5 Gew.-% Cetostearylalkohol, 3,5 Gew.-% Polyoxyethylenglycerylmonostearat, 1,5 Gew.-% Glycerylmonostearat, 5,0 Gew.-% 1,3-Butylenglycol, 5,0 Gew.-% Glycerin, 5,0 Gew.-% eines Gemischs von Squalan, Macadamianussöl, Olivenöl und Oliwax, 0,12 Gew.-% Betamethasonvalerat, 0,3 Gew.-% Konservierungsmittel, 0,2 Gew.-% Carboxyvinylpolymer und Wasser; oder

(11)

0,6 Gew.-% Pseudoceramid PC-9SP, 0,2 Gew.-% Cholesterin, 1,5 Gew.-% Stearinsäure, 3,5 Gew.-% Cetostearylalkohol, 3,5 Gew.-% Polyoxyethylenglycerylmonostearat, 1,5 Gew.-% Glycerylmonostearat, 5,0 Gew.-% 1,3-Butylenglycol, 5,0 Gew.-% Glycerin, 5,0 Gew.-% eines Gemischs von Squalan, Macadamianussöl, Olivenöl und Oliwax, 0,05 Gew.-% Clobetasolpropionat, 0,3 Gew.-% Konservierungsmittel, 0,2 Gew.-% Carboxyvinylpolymer und Wasser; oder

(12)

0,6 Gew.-% Pseudoceramid PC-9SP, 0,2 Gew.-% Cholesterin, 1,5 Gew.-% Stearinsäure, 3,5 Gew.-% Cetostearylalkohol, 3,5 Gew.-% Polyoxyethylenglycerylmonostearat, 1,5 Gew.-% Glycerylmonostearat, 5,0 Gew.-% 1,3-Butylenglycol, 5,0 Gew.-% Glycerin, 5,0 Gew.-% eines Gemischs von Squalan, Macadamianussöl, Olivenöl und Oliwax, 1,0 Gew.-% Tretinoin, 0,3 Gew.-% Konservierungsmittel, 0,2 Gew.-% Carboxyvinylpolymer und Wasser; oder

(13)

0,6 Gew.-% Pseudoceramid PC-9SP, 0,2 Gew.-% Cholesterin, 1,5 Gew.-% Stearinsäure, 3,5 Gew.-% Cetostearylalkohol, 3,5 Gew.-% Polyoxyethylenglycerylmonostearat, 1,5 Gew,-% Glycerylmonostearat, 5,0 Gew.-% 1,3-Butylenglycol, 5,0 Gew.-% Glycerin, 5,0 Gew.-% eines Gemischs von Squalan, Macadamianussöl, Olivenöl, und Oliwax, 0,5 Gew.-% Linolsäure, 0,3 Gew.-% Konservierungsmittel, 0,2 Gew.-% Carboxyvinylpolymer und Wasser; oder

(14)

0,6 Gew.-% Ceramid III, 0,2 Gew.-% Cholesterin, 1,5 Gew.-% Stearinsäure, 3,5 Gew.-% Cetostearylalkohol, 3,5 Gew.-% Polyoxyethylenglycerylmonostearat, 1,5 Gew.-% Glycerylmonostearat, 5,0 Gew.-% 1,3-Butylenglycol, 5,0 Gew.-% Glycerin, 5,0 Gew.-% eines Gemischs von Squalan, Macadamianussöl, Olivenöl und Oliwax, 0,5 Gew.-% Hydrocortison, 0,3 Gew.-% Konservierungsmittel, 0,2 Gew.-% Carboxyvinylpolymer und Wasser; oder

(15)

0,6 Gew.-% Ceramid III, 0,2 Gew.-% Cholesterin, 1,5 Gew.-% Stearinsäure, 3,5 Gew.-% Cetostearylalkohol, 3,5 Gew.-% Polyoxyethylenglycerylmonostearat, 1,5 Gew.-% Glycerylmonostearat, 5,0 Gew.-% 1,3-Butylenglycol, 5,0 Gew.-% Glycerin, 5,0 Gew.-% eines Gemischs von Squalan, Macadamianussöl, Olivenöl und Oliwax, 0,12 Gew.-% Betamethasonvalerat, 0,3 Gew.-% Konservierungsmittel, 0,2 Gew.-% Carboxyvinylpolymer und Wasser; oder

(16)

0,6 Gew.-% Ceramid III, 0,2 Gew.-% Cholesterin, 1,5 Gew.-% Stearinsäure, 3,5 Gew.-% Cetostearylalkohol, 3,5 Gew.-% Polyoxyethylenglycerylmonostearat, 1,5 Gew.-% Glycerylmonostearat, 5,0 Gew.-% 1,3-Butylenglycol, 5,0 Gew.-% Glycerin, 5,0 Gew.-% eines Gemischs von Squalan, Macadamianussöl, Olivenöl und Oliwax, 0,05 Gew.-% Clobeatsolpropionat, 0,3 Gew.-% Konservierungsmittel, 0,2 Gew.-% Carboxyvinylpolymer und Wasser; oder

(17)
0,6 Gew.-% Ceramid III, 0,2 Gew.-% Cholesterin, 1,5 Gew.-% Stearinsäure, 3,5 Gew.-% Cetostearylalkohol, 3,5 Gew.-% Polyoxyethylenglycerylmonostearat, 1,5 Gew.-% Glycerylmonostearat, 5,0 Gew.-% 1,3-Butylenglycol, 5,0 Gew.-% Glycerin, 5,0 Gew.-% eines Gemischs von Squalan, Macadamianussöl, Olivenöl und Oliwax, 1,0 Gew.-% Tretinoin, 0,3 Gew.-% Konservierungsmittel, 0,2 Gew.-% Carboxyvinylpolymer und Wasser; oder
(18)
0,6 Gew.-% Ceramid III, 0,2 Gew.-% Cholesterin, 1,5 Gew.-% Stearinsäure, 3,5 Gew.-% Cetostearylalkohol, 3,5 Gew.-% Polyoxyethylenglycerylmonostearat, 1,5 Gew.-% Glycerylmonostearat, 5,0 Gew.-% 1,3-Butylenglycol, 5,0 Gew.-% Glycerin, 5,0 Gew.-% eines Gemischs von Squalan, Macadamianussöl, Olivenöl und Oliwax, 0,5 Gew.-% Linolsäure, 0,3 Gew.-% Konservierungsmittel, 0,2 Gew.-% Carboxyvinylpolymer und Wasser.

## Revendications

**1.** Emulsion multi-lamellaire (MLE) pour la stabilisation d'un ingrédient oléophile utile sur le plan dermatologique, la MLE comprenant :

un composant à base de lipides épidermiques comprenant un ingrédient lipidique situé entre les cellules du stratum corneum ;
un composant à base d'huile; et
un composant à base d'un émulsifiant qui est un tensioactif non ionique,
dans laquelle la MLE a une composition huileuse représentée par l'Equation 1 :

[Equation 1]

$$\text{composition huileuse} = \frac{\text{contenu du composant à base d'huile}}{\text{contenu du composant à base d'un émulsifiant} + \text{contenu du composant à base de lipides épidermiques}} = 0.3 - 1.0$$

où le composant à base de lipides épidermiques est la composition comprenant :
un pseudocéramide ou un céramide naturel
un cholestérol
de l'acide stéarique et
un alcool cétostéarylique
dans laquelle la MLE comprend :

(1)
0,5 % en poids de pseudocéramide PC-9S, 0,25 % en poids de cholestérol, 1,0 % en poids d'acide stéarique, 2,0 % en poids d'alcool cétostéarylique, 3,0 % en poids de monostéarate de polyoxyéthylène, 2 % en poids de monostéarate de glycéryle, 5,0 % en poids d'un mélange d'huile d'olive, de paraffine liquide, de vaseline et de cire de paraffine, 5,0 % en poids de 1,3-butylène glycol, 0,3 % en poids de palmitate de rétinol, 0,2 % en poids de polymère carboxylvinylique et de l'eau ; ou
(2) 0,8 % en poids de pseudocéramide PC-9S, 0,4 % en poids de cholestérol, 2,8 % en poids d'acide stéarique, 2,0 % en poids d'alcool cétostéarylique, 3,0 % en poids de monostéarate de polyoxyéthylène glycéryle, 1 % en poids de monostéarate de glycéryle, 5,0 % en poids d'un mélange d'huile d'olive, de paraffine liquide, de vaseline et de cire de paraffine, 5,0 % en poids de 1,3-butylène glycol, 0,3 % en poids de palmitate de rétinol, 0,2 % en poids de polymère carboxylvinylique et de l'eau ; ou
(3) 1,0 % en poids de pseudocéramide PC-9S, 0,1 % en poids de cholestérol, 3,5 % en poids d'acide stéarique, 1,6 % en poids d'alcool cétostéarylique, 2,2 % en poids de monostéarate de polyoxyéthylène sorbitane, 0,5 % en poids de monostéarate de sorbitane, 1,2 % en poids de monostéarate de glycéryle, 5,0 % en poids d'un mélange d'huile d'olive, de paraffine liquide, de vaseline et de cire de paraffine, 5,0 % en poids de 1,3-butylène glycol, 0,3 % en poids de palmitate de rétinol, 0,2 % en poids de polymère carboxylvinylique et de l'eau ; ou
(4)
0,4 % en poids de pseudocéramide PC-9S, 0,15 % en poids de cholestérol, 1,0 % en poids d'acide stéarique, 2,0 % en poids d'alcool cétostéarylique, 2,0 % en poids de monostéarate de polyoxyéthylène glycéryle, 1,0 % en poids de monostéarate de glycéryle, 4,0 % en poids d'un mélange d'huile d'olive, de paraffine liquide, de vaseline et de cire de paraffine, 5,0 % en poids de 1,3-butylène glycol, 0,3 % en poids de palmitate de rétinol, 0,2 % en poids de polymère carboxylvinylique et de l'eau ; ou

(5)

0,5 % en poids de pseudocéramide PC-9S, 0,15 % en poids de cholestérol, 1,2 % en poids d'acide stéarique, 2,5 % en poids d'alcool cétostéarylique, 2,5 % en poids de monostéarate de polyoxyéthylène glycéryle, 1,2 % en poids de monostéarate de glycéryle, 3,0 % en poids d'un mélange d'huile d'olive, de paraffine liquide, de vaseline et de cire de paraffine, 5,0 % en poids de 1,3-butylène glycol, 0,3 % en poids de palmitate de rétinol, 0,2 % en poids de polymère carboxylvinylique et de l'eau ; ou

(6)

0,6 % en poids de pseudocéramide PC-9S, 0,2 % en poids de cholestérol, 1,5 % en poids d'acide stéarique, 3,0 % en poids d'alcool cétostéarylique, 3,0 % en poids de monostéarate de polyoxyéthylène glycéryle, 1,5% en poids de monostéarate de glycéryle, 5,0 % en poids d'un mélange d'huile d'olive, de paraffine liquide, de vaseline et de cire de paraffine, 5,0 % en poids de 1,3-butylène glycol, 0,3 % en poids de palmitate de rétinol, 0,2 % en poids de polymère carboxylvinylique et de l'eau ; ou

(7)

0,8 % en poids de pseudocéramide PC-9S, 0,3 % en poids de cholestérol, 1,8 % en poids d'acide stéarique, 3,5 % en poids d'alcool cétostéarylique, 3,2 % en poids de m-nostéarate de polyoxyéthylène glycéryle, 1,8 % en poids de monostéarate de glycéryle, 8,0 % en poids d'un mélange d'huile d'olive, de paraffine liquide, de vaseline et de cire de paraffine, 5,0 % en poids de 1,3-butylène glycol, 0,3 % en poids de palmitate de rétinol, 0,2 % en poids de polymère carboxylvinylique et de l'eau ; ou

(8)

1,0 % en poids de pseudocéramide PC-9S, 0,3 % en poids de cholestérol, 2,0 % en poids d'acide stéarique, 4,0 % en poids d'alcool cétostéarylique, 4,0 % en poids de monostéarate de polyoxyéthylène glycéryle, 2,0 % en poids de monostéarate de glycéryle, 13,0 % en poids d'un mélange d'huile d'olive, de paraffine liquide, de vaseline et de cire de paraffine, 5,0 % en poids de 1,3-butylène glycol, 0,3 % en poids de palmitate de rétinol, 0,2 % en poids de polymère carboxylvinylique et de l'eau ; ou

(9)

0,6 % en poids de pseudocéramide PC-9S, 0,2 % en poids de cholestérol, 1,5 % en poids d'acide stéarique, 3,5 % en poids d'alcool cétostéarylique, 3,5 % en poids de monostéarate de polyoxyéthylène glycéryle, 1,5 % en poids de monostéarate de glycéryle, 5,0 % en poids de 1,3-butylène glycol, 5,0 % en poids de glycérine, 5,0 % en poids d'un mélange de squalane, d'huile de noix de macadamia, d'huile d'olive et d'Oliwax, 0,5 % en poids d'hydrocortisone, 0,3 % en poids d'agent conservateur, 0,2 % en poids de polymère carboxylvinylique et de l'eau ; ou

(10)

0,6 % en poids de pseudocéramide PC-9S, 0,2 % en poids de cholestérol, 1,5 % en poids d'acide stéarique, 3,5 % en poids d'alcool cétostéarylique, 3,5 % en poids de monostéarate de polyoxyéthylène glycéryle, 1,5 % en poids de monostéarate de glycéryle, 5,0 % en poids de 1,3-butylène glycol, 5,0 % en poids de glycérine, 5,0 % en poids d'un mélange de squalane, d'huile de noix de macadamia, d'huile d'olive et d'Oliwax, 0,12 % en poids de valérate de bétaméthasone, 0,3 % en poids d'agent conservateur, 0,2 % en poids de polymère carboxylvinylique et de l'eau ; ou

(11)

0,6 % en poids de pseudocéramide PC-9S, 0,2 % en poids de cholestérol, 1,5 % en poids d'acide stéarique, 3,5 % en poids d'alcool cétostéarylique, 3,5 % en poids de monostéarate de polyoxyéthylène glycéryle, 1,5 % en poids de monostéarate de glycéryle, 5,0 % en poids de 1,3-butylène glycol, 5,0 % en poids de glycérine, 5,0 % en poids d'un mélange de squalane, d'huile de noix de macadamia, d'huile d'olive et d'Oliwax, 0,05 % en poids de propionate de clobétasol, 0,3 % en poids d'agent conservateur, 0,2 % en poids de polymère carboxylvinylique et de l'eau ; ou

(12)

0,6 % en poids de pseudocéramide PC-9S, 0,2 % en poids de cholestérol, 1,5 % en poids d'acide stéarique, 3,5 % en poids d'alcool cétostéarylique, 3,5 % en poids de monostéarate de polyoxyéthylène glycéryle, 1,5 % en poids de monostéarate de glycéryle, 5,0 % en poids de 1,3-butylène glycol, 5,0 % en poids de glycérine, 5,0 % en poids d'un mélange de squalane, d'huile de noix de macadamia, d'huile d'olive et d'Oliwax, 1,0 % en poids de trétinoïne, 0,3 % en poids d'agent conservateur, 0,2 % en poids de polymère carboxylvinylique et de l'eau ; ou

(13)

0,6 % en poids de pseudocéramide PC-9S, 0,2 % en poids de cholestérol, 1,5 % en poids d'acide stéarique, 3,5 % en poids d'alcool cétostéarylique, 3,5 % en poids de monostéarate de polyoxyéthylène glycéryle, 1,5 % en poids de monostéarate de glycéryle, 5,0 % en poids de 1,3-butylène glycol, 5,0 % en poids de glycérine, 5,0 % en poids d'un mélange de squalane, d'huile de noix de macadamia, d'huile d'olive et d'Oliwax, 0,5 % en poids d'acide linoléique, 0,3 % en poids d'agent conservateur, 0,2 % en poids de polymère carboxylvinylique et de l'eau ; ou

(14)

0,6 % en poids de céramide III, 0,2 % en poids de cholestérol, 1,5 % en poids d'acide stéarique, 3,5 % en poids d'alcool cétostéarylique, 3,5 % en poids de monostéarate de polyoxyéthylène glycéryle, 1,5 % en poids de monostéarate de glycéryle, 5,0 % en poids de 1,3-butylène glycol, 5,0 % en poids de glycérine, 5,0 % en poids d'un mélange de squalane, d'huile de noix de macadamia, d'huile d'olive et d'Oliwax, 0,5 % en poids d'hydrocortisone, 0,3 % en poids d'agent conservateur, 0,2 % en poids de polymère carboxylvinylique et de l'eau ; ou

(15)

0,6 % en poids de céramide III, 0,2 % en poids de cholestérol, 1,5 % en poids d'acide stéarique, 3,5 % en poids d'alcool cétostéarylique, 3,5 % en poids de monostéarate de polyoxyéthylène glycéryle, 1,5 % en poids de monostéarate de glycéryle, 5,0 % en poids de 1,3-butylène glycol, 5,0 % en poids de glycérine, 5,0 % en poids d'un mélange de squalane, d'huile de noix de macadamia, d'huile d'olive et d'Oliwax, 0,12 % en poids de valérate de bétaméthasone, 0,3 % en poids d'agent conservateur, 0,2 % en poids de polymère carboxylvinylique et de l'eau ; ou

(16)

0,6 % en poids de céramide III, 0,2 % en poids de cholestérol, 1,5 % en poids d'acide stéarique, 3,5 % en poids d'alcool cétostéarylique, 3,5 % en poids de monostéarate de polyoxyéthylène glycéryle, 1,5 % en poids de monostéarate de glycéryle, 5,0 % en poids de 1,3-butylène glycol, 5,0 % en poids de glycérine, 5,0 % en poids d'un mélange de squalane, d'huile de noix de macadamia, d'huile d'olive et d'Oliwax, 0,05 % en poids de propionate de clobétasol, 0,3 % en poids d'agent conservateur, 0,2 % en poids de polymère carboxylvinylique et de l'eau ; ou

(17)

0,6 % en poids de céramide III, 0,2 % en poids de cholestérol, 1,5 % en poids d'acide stéarique, 3,5 % en poids d'alcool cétostéarylique, 3,5 % en poids de monostéarate de polyoxyéthylène glycéryle, 1,5 % en poids de monostéarate de glycéryle, 5,0 % en poids de 1,3-butylène glycol, 5,0 % en poids de glycérine, 5,0 % en poids d'un mélange de squalane, d'huile de noix de macadamia, d'huile d'olive et d'Oliwax, 1,0 % en poids de trétinoïne, 0,3 % en poids d'agent conservateur, 0,2 % en poids de polymère carboxylvinylique et de l'eau ; ou

(18)

0,6 % en poids de céramide III, 0,2 % en poids de cholestérol, 1,5 % en poids d'acide stéarique, 3,5 % en poids d'alcool cétostéarylique, 3,5 % en poids de monostéarate de polyoxyéthylène glycéryle, 1,5 % en poids de monostéarate de glycéryle, 5,0 % en poids de 1,3-butylène glycol, 5,0 % en poids de glycérine, 5,0 % en poids d'un mélange de squalane, d'huile de noix de macadamia, d'huile d'olive et d'Oliwax, 0,5 % en poids d'acide linoléique, 0,3 % en poids d'agent conservateur, 0,2 % en poids de polymère carboxylvinylique et de l'eau.

【Fig 1】

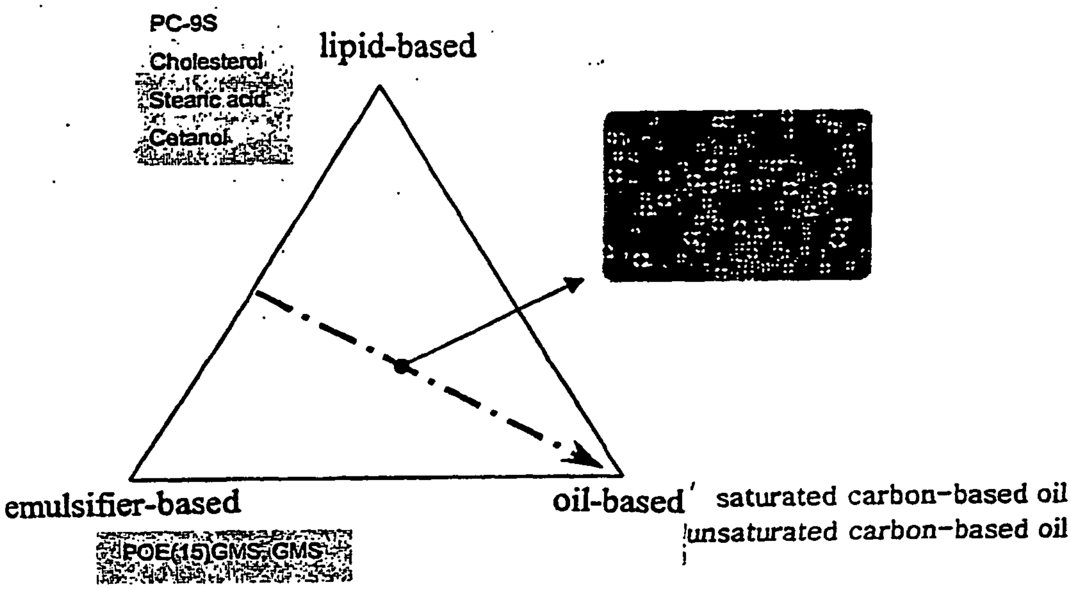
PC-9S
Cholesterol
Stearic acid
Cetanol
lipid-based
emulsifier-based
POE(15)GMS, GMS
oil-based
saturated carbon-based oil
unsaturated carbon-based oil

【Fig 2a】

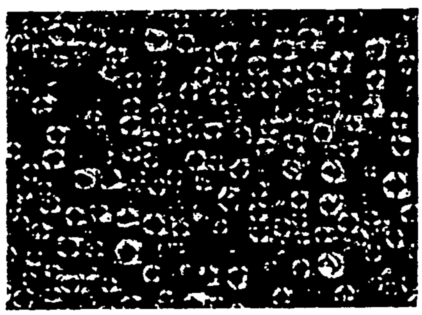

【Fig 2b】

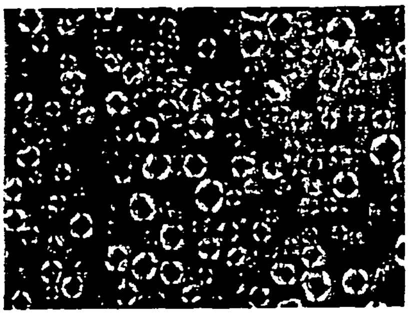

【Fig 3】

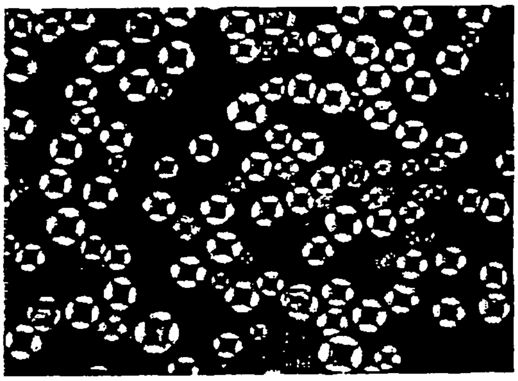

【Fig 4】

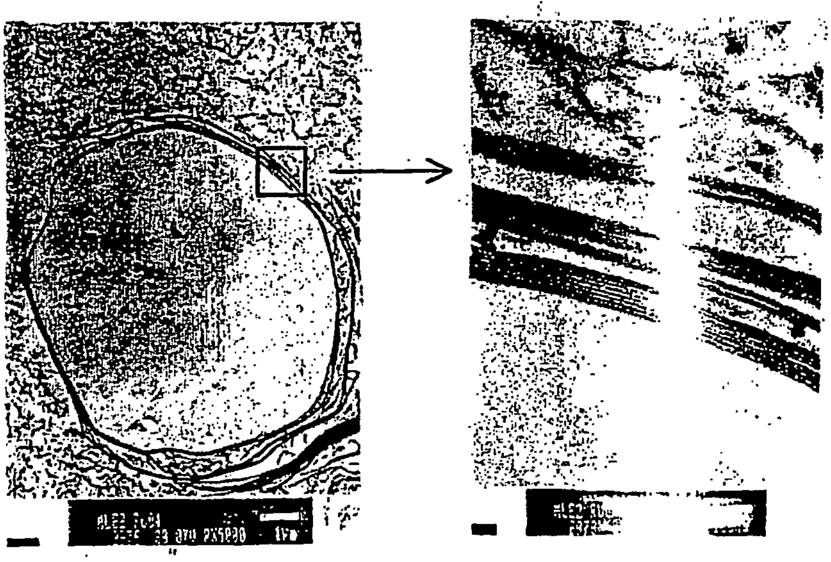

【Fig 5a】

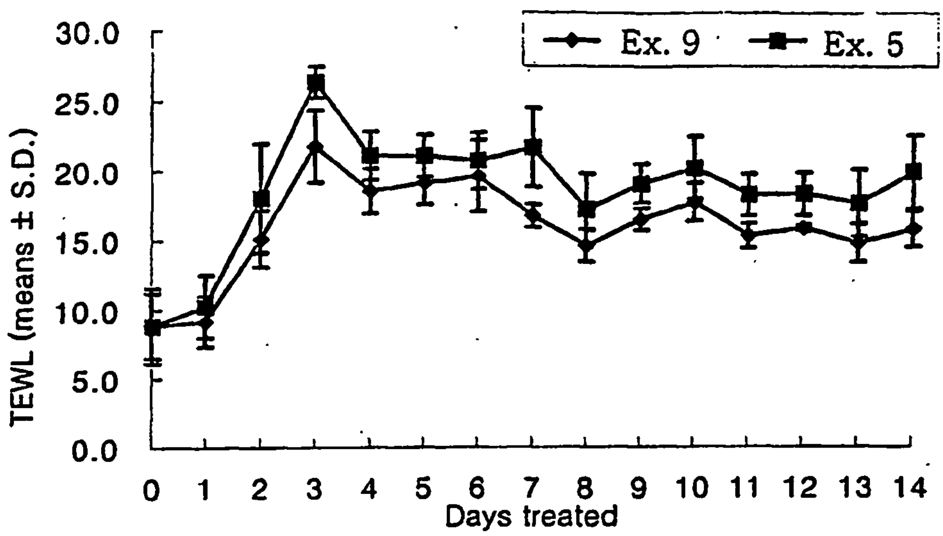
Ex. 9
Ex. 5
30.0
25.0
20.0
15.0
10.0
5.0
0.0
TEWL (means ± S.D.)
0 1 2 3 4 5 6 7 8 9 10 11 12 13 14
Days treated

【Fig 5b】

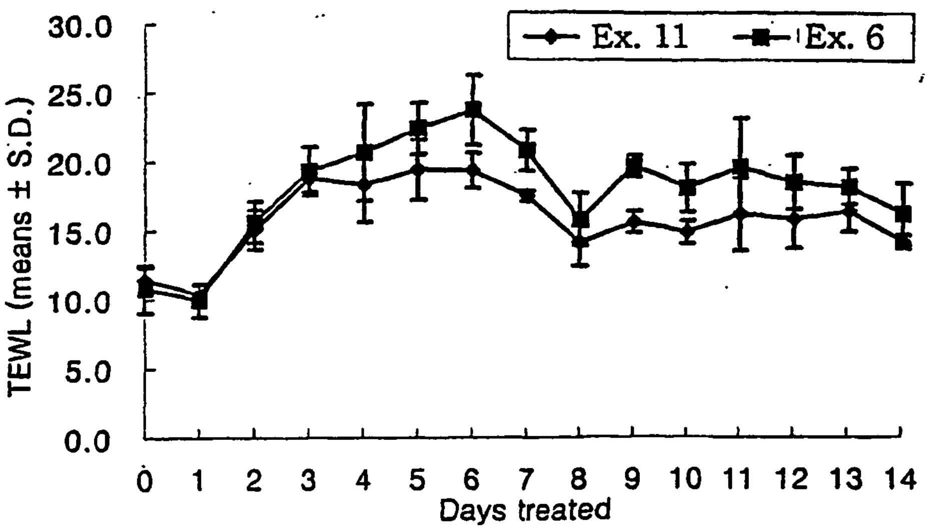
Ex. 11
Ex. 6
30.0
25.0
20.0
15.0
10.0
5.0
0.0
TEWL (means ± S.D.)
0 1 2 3 4 5 6 7 8 9 10 11 12 13 14
Days treated

【Fig 6a】

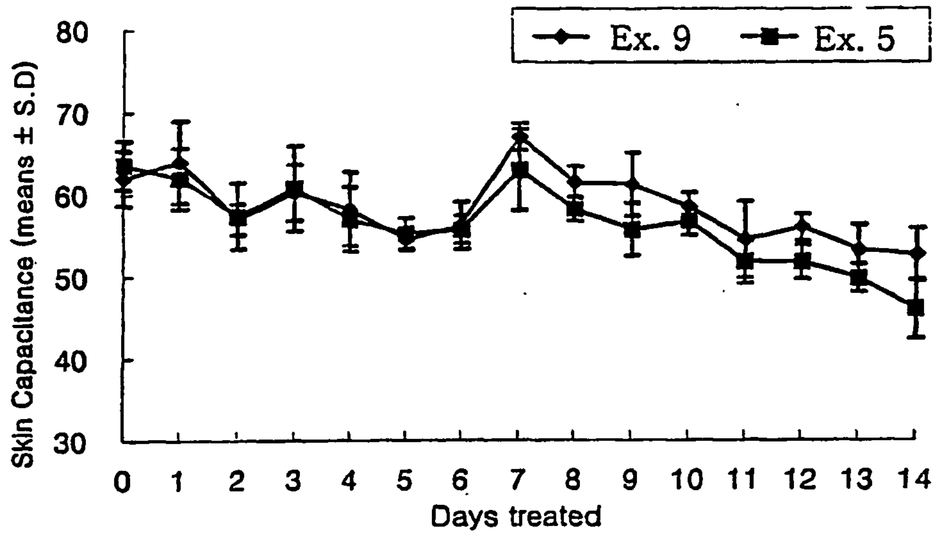
Ex. 9
Ex. 5
Skin Capacitance (means ± S.D)
80
70
60
50
40
30
0 1 2 3 4 5 6 7 8 9 10 11 12 13 14
Days treated

【Fig 6b】

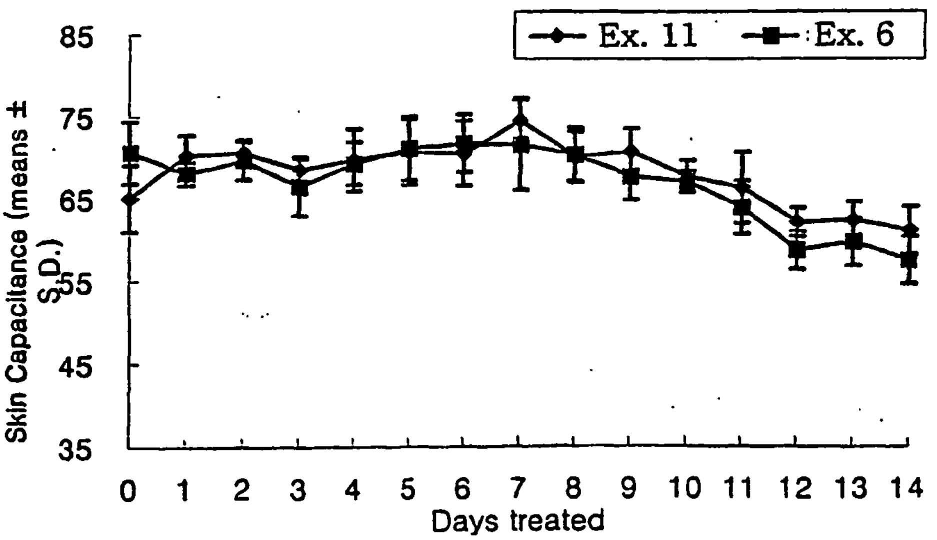
Ex. 11
Ex. 6
Skin Capacitance (means ± S.D.)
85
75
65
55
45
35
0 1 2 3 4 5 6 7 8 9 10 11 12 13 14
Days treated

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0875232 A
- US 5985177 A
- US 5980917 A
- KR 2000055082

### Non-patent literature cited in the description

- *Skin Research and Technology,* 2001, vol. 7, 73-77